# EUROPEAN PATENT SPECIFICATION

(11) **EP 4 419 905 B1**
(45) Date of publication and mention of the grant of the patent: **07.10.2026**
(21) Application number: 22805908.5
(22) Date of filing: 20.10.2022
(51) Int. Cl.: G01N 33/00

(54) **SYSTEM AND METHOD EMPLOYING A GROUP OF SENSORS FOR DETECTING SUBSTANCES IN A GAS, AND CORRESPONDING COMPUTER PROGRAM**
SYSTEM UND VERFAHREN MIT EINER GRUPPE VON SENSOREN ZUM NACHWEIS VON SUBSTANZEN IN EINEM GAS UND ENTSPRECHENDES COMPUTERPROGRAMM
SYSTÈME ET PROCÉDÉ UTILISANT UN GROUPE DE CAPTEURS POUR DÉTECTER DES SUBSTANCES DANS UN GAZ, ET PROGRAMME INFORMATIQUE CORRESPONDANT

(30) Priority: 23.10.2021 FI 20216105
(43) Date of publication of application: 28.08.2024
(73) Proprietor: Orava, Risto, 04420 Järvenpää (FI)
(72) Inventor: Orava, Risto, 04420 Järvenpää (FI)
(74) Representative: Väänänen, Janne Kalervo
(86) International application number: PCT/FI2022/050699
(87) International publication number: WO 2023/067248

(56) References cited:
- WO-A1-2009/066293
- WO-A1-2014/111611
- WO-A1-2017/216794
- SIMON M SCOTT ET AL: "Data analysis for electronic nose systems", MICROCHIMICA ACTA ; AN INTERNATIONAL JOURNAL ON MICRO AND TRACEANALYSIS, SPRINGER-VERLAG, VI, vol. 156, no. 3-4, 29 September 2006 (2006-09-29), pages 183 - 207, XP019461633, ISSN: 1436-5073, DOI: 10.1007/S00604-006-0623-9

## Description

### Technical field

The disclosure relates to a system for detecting one or more substances, such as volatile organic compounds "VOC", carried by air or other gas. The system can be for example a gas sample analyzer system for detecting substances contained by a sample of air or other gas. Furthermore, the disclosure relates to a method for detecting one or more substances carried by air or other gas. Furthermore, the disclosure relates to a computer program for detecting one or more substances carried by air or other gas.

### Background

In many cases, there is a need to detect substances carried by air or other gas. For example, detection of substances such as e.g. volatile organic compounds "VOC" contained by air and/or other gases exuded by a living organism, such as an animal or plant, has become a subject of significant research interest because of the rich information that can be extracted from the air and/or other gases exuded by the living organism. This opens a wide variety of potential applications in the field of detection and identification of different states of living organisms. For example, a viral infection in a living organism has a characteristic VOC profile in air or other gas exuded by the living organism. This VOC profile can be determined by classifying samples of air or other gas collected from infected, PCR-positive, and non-infected, PCR negative, living organisms. The PCR is the acronym of the polymerase chain reaction test that is capable of detecting genetic material from a specific organism, such as the H3N8 or H3N2 virus. In many cases, the PCR test is used as the 'gold plated' reference to define the infected, PCR positives, and not-infected, PCR negatives. Other exemplifying applications based on detection of substances carried by air or other gas are: detecting toxins and/or other hazardous elements such as carbon monoxide, alcohol detection passively or actively, qualitative and quantitative analysis in petroleum industry, explosive detection, detection for environmental applications, quality control in industry such as automotive industry, discriminating between clean and contamination in milking systems, cosmetic raw materials analysis, space applications, and chemical warfare detection.

A system for detecting substances of interest in air, specifically disease markers in exhaled breath, is known from WO2017216794, which uses a group of sensors with different and overlapping sensitivities for different analytes, and employs a pattern recognition algorithm to determine the reference pattern closest, i.e. with the smallest distance to, the measured sensor value pattern.

In the light of the above-mentioned, there is a need for devices and systems for detecting substances carried by air or other gas. An inherent challenge related to devices and systems for detecting substances carried by air or other gas is that the air or the other gas may contain substances which may significantly disturb and complicate the detection of the substances of interest.

### Summary

The following presents a simplified summary in order to provide a basic understanding of some aspects of various invention embodiments. The summary is not an extensive overview of the invention. It is neither intended to identify key or critical elements of the invention nor to delineate the scope of the invention. The following summary merely presents some concepts of the invention in a simplified form as a prelude to a more detailed description of exemplifying embodiments of the invention.

In accordance with the invention, there is provided a new system for detecting one or more substances carried by air or other gas. The system can be for example a gas sample analyzer system for detecting one or more substances contained by a gas sample. The system can be implemented as a single device, or the system may have a distributed architecture so that the system comprises a measurement device and a server located in a data network and communicatively connected to the measurement device.

A system according to the invention comprises:
- a group of sensors responsive to many substances to produce a group of sensor values, the sensors having crossing acceptances for the substances so that different ones of the sensors have maximum acceptances for different ones of the substances, and
- a processing system configured to:
- select first subgroups from the group of sensor values, the first subgroups being different from each other,
- compute a group of first indicator values so that each of the first indicator values is related to a corresponding one of the first subgroups and is a first pre-determined function of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- select a second subgroup from the group of the first indicator values in accordance with a first predetermined selection criterion, and
- compute a distance function between a measurement pattern constituted by at least the points corresponding to the first indicator values belonging to the second subgroup and a reference pattern corresponding to one or more of the substances, a value of the distance function being indicative of presence or absence of the one or more of the substances.

In this document, the 'acceptance' describes how strongly a sensor value produced by a sensor under consideration changes linearly or non-linearly when an amount of substance, e.g. a VOC, under consideration changes in a gas space next to an active surface of the sensor. In this document, the 'crossing acceptances' means that the sensors are multispectral so that each sensor is responsive not only to one substance but also to one or more other substances to which one or more of the other sensors is/are responsive, too.

The above-mentioned reference pattern corresponds advantageously to a mixture of substances, for example a mixture of VOCs. In an application for detecting a viral infection, the mixture of VOCs is advantageously a VOC profile characteristic to the viral infection. In empirical tests, it has turned out that a mixture of substances can be detected more reliably by comparing the measurement pattern to a reference pattern corresponding to the mixture than by comparing the measurement pattern to many component-specific reference patterns one-by-one, wherein each component-specific reference pattern corresponds to a single component of the mixture.

The reference pattern can be based on empirical tests where air or other gas that is known to contain one or more substances of interest is directed to the group of sensors. The reference pattern can be formed based on the measurement pattern obtained in an empirical test of the kind mentioned above. More advantageously, the reference pattern is formed based on many measurement patterns obtained in many empirical tests of the kind mentioned above. In an application for detecting viral infections, the air or other gas that is used in the empirical tests can be samples of air or other gas collected from infected living organisms such as e.g. animals. In cases where the outcome of the detection is a selection from a finite number of alternatives, e.g. yes/no decision that is a selection from two alternatives, the processing system is advantageously configured to compare the measurement pattern to many reference patterns and select the alternative corresponding to the nearest one of the reference patterns. In an application for detecting viral infections, the air or other gas that is used in the empirical tests for producing the reference pattern related to infected cases can be samples of air or other gas collected from infected living organisms and the air or other gas that is used in the empirical tests for producing the reference pattern related to non-infected cases can be samples of air or other gas collected from non-infected living organisms.

In accordance with the invention, there is also provided a new method for detecting one or more substances carried by air or other gas. A method according to the invention comprises:
- producing a group of sensor values with a group of sensors responsive to many substances, the sensors having crossing acceptances for the substances so that different ones of the sensors have maximum acceptances for different ones of the substances,
- selecting first subgroups from the group of sensor values, the first subgroups being different from each other,
- computing a group of first indicator values so that each of the first indicator values is related to a corresponding one of the first subgroups and is a first pre-determined function of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- selecting a second subgroup from the group of the first indicator values in accordance with a first predetermined selection criterion, and
- computing a distance function between a measurement pattern constituted by at least the points corresponding to the first indicator values belonging to the second subgroup and a reference pattern corresponding to one or more of the substances, a value of the distance function being indicative of presence or absence of the one or more of the substances.

In accordance with the invention, there is also provided a new computer program for detecting one or more substances carried by air or other gas. A computer program according to the invention comprises computer executable instructions for controlling a programmable processing system to:
- receive a group of sensor values from a group of sensors responsive to many substances, the sensors having crossing acceptances for the substances so that different ones of the sensors have maximum acceptances for different ones of the substances,
- select first subgroups from the group of sensor values, the first subgroups being different from each other,
- compute a group of first indicator values so that each of the first indicator values is related to a corresponding one of the first subgroups and is a first pre-determined function of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,

- select a second subgroup from the group of the first indicator values in accordance with a first predetermined selection criterion, and
- compute a distance function between a measurement pattern constituted by at least the points corresponding to the first indicator values belonging to the second subgroup and a reference pattern corresponding to one or more of the substances, a value of the distance function being indicative of presence or absence of the one or more of the substances.

A computer program product can be provided which comprises a non-transitory computer readable medium, e.g. a compact disc "CD", encoded with a computer program according to the invention.

Exemplifying and non-limiting embodiments are described in accompanied dependent claims.

Various exemplifying and non-limiting embodiments both as to constructions and to methods of operation, together with additional objects and advantages thereof, will be best understood from the following description of specific exemplifying and non-limiting embodiments when read in conjunction with the accompanying drawings. The verbs "to comprise" and "to include" are used in this document as open limitations that neither exclude nor require the existence of unrecited features. The features recited in dependent claims are mutually freely combinable unless otherwise explicitly stated.

Furthermore, it is to be understood that the use of "a" or "an", i.e. a singular form, throughout this document does not exclude a plurality.

### Brief description of the figures

Exemplifying and non-limiting embodiments and their advantages are explained in greater detail below in the sense of examples and with reference to the accompanying drawings, in which:
figure 1 shows a functional block-diagram of a system according to an exemplifying and non-limiting embodiment for detecting one or more of substances carried by air or other gas,
figure 2 illustrates data processing in a system according to an exemplifying and non-limiting embodiment for detecting one or more of substances carried by air or other gas, and
figure 3 shows a flowchart of a method according to an exemplifying and non-limiting embodiment for detecting one or more of substances carried by air or other gas.

### Description of exemplifying and non-limiting embodiments

The specific examples provided in the description below should not be construed as limiting the scope and/or the applicability of the accompanied claims. Lists and groups of examples provided in the description are not exhaustive unless otherwise explicitly stated.

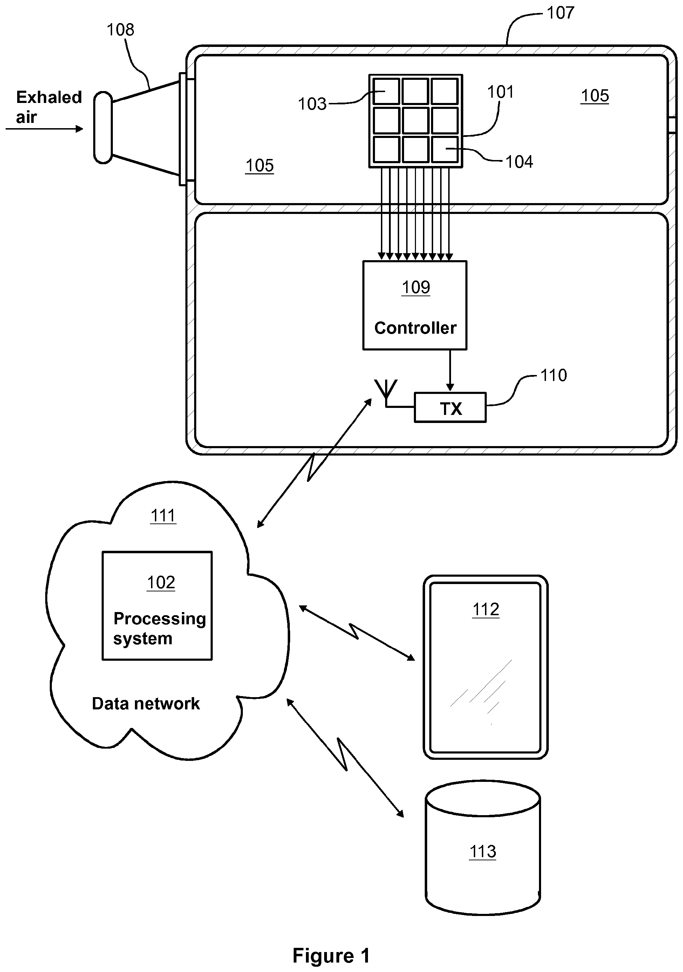

Figure 1 shows a functional block-diagram of a system according to an exemplifying and non-limiting embodiment for detecting one or more of substances, e.g. VOCs, carried by air or other gas. In this exemplifying case, the system is a gas sample analyzer system that has a distributed architecture. The system comprises a measurement device 107 and a processing system 102 located in a data network 111. The processing system 102 is communicatively connected to the measurement device 107. Furthermore, the processing system 102 can be communicatively connected to other devices such as a mobile phone 112 of a user and/or a data storage device 113. It is, however, also possible that the system has a centralized architecture so that the processing system 102 and the measurement device 107 constitute a single apparatus.

The measurement device 107 comprises a body section constituting a gas collection cavity 105 for receiving a sample of air or other gas. The sample of gas is received via an inlet element 108. The inlet element 108 is advantageously a changeable component of the measurement device 107. The measurement device 107 comprises a group 101 of sensors that is at least partially inside the gas collection cavity 105. In this exemplifying case, the group 101 of sensors is a 3×3 sensor array. In figure 1, two of the sensors are denoted with references 103 and 104. The sensors are responsive to many substances, e.g. VOCs, to produce a group of sensor values. The sensors have crossing acceptances for the substances so that different ones of the sensors have maximum acceptances for different ones of the substances. The sensors can be for example metal oxide "MOX" sensors or some other suitable gas sensors. The maximum acceptances of the metal oxide sensors can be arranged to correspond to different substances by controlling temperatures of heater plates of the metal oxide sensors so that the heater plates of different ones of the metal oxide sensors operate at different temperatures. It is to be noted that the invention is not limited to any specific sensor type. In an exemplifying case in which the system is used for detecting a viral infection, the sensors are advantageously responsive to the following VOCs: acetone, butyraldehyde, ethyl butanoate, and isopropanol.

The measurement device 107 comprises a controller 109 for producing the sensor values by sampling output signals of the sensors. The sampling rate can be for example 1 Hz. In an exemplifying case in which the sensors are MOX sensors, the output signals of the sensors are indicative of electrical resistances of active parts of the sensors. The measurement device 107 comprises a radio transmitter 110 configured to transmit the sensor values to the processing system 102 located in the data network 111.

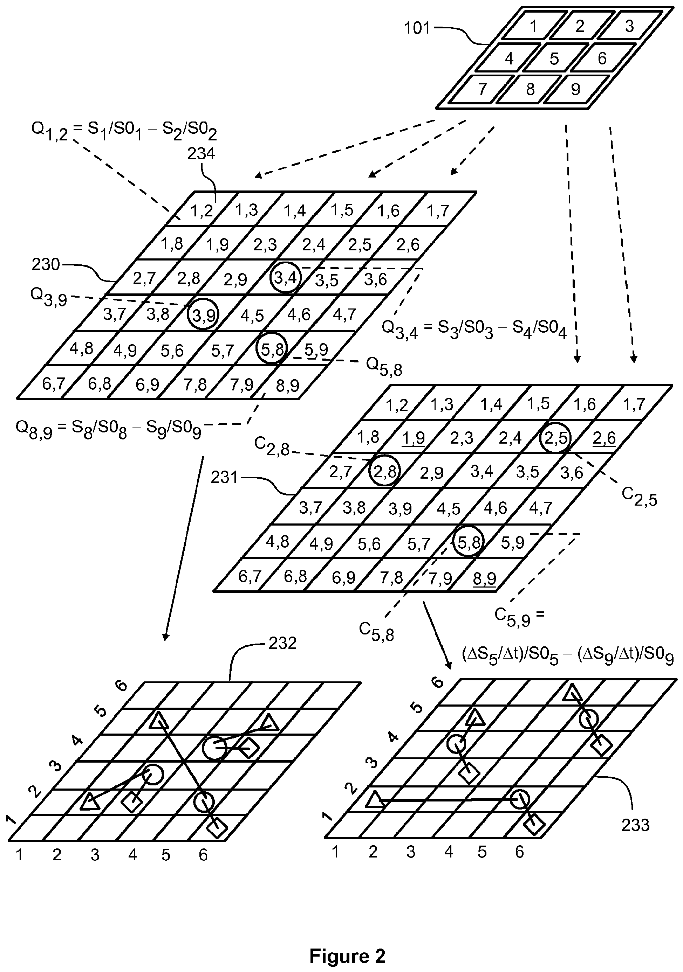

An exemplifying functionality of the processing system 102 is explained below with reference to figure 2. For the sake of illustrative purposes, the sensors of the group 101 of sensors are denoted with numbers 1, 2, 3, 4, 5, 6, 7, 8, and 9. The processing system 102 is configured to select first subgroups from the group of sensor values S₁...S₉ so that the first subgroups are different from each other. In this case, each first subgroup contains two sensor values Sᵢ and Sⱼ where i ≠ j. Therefore, the number of subgroups is 9 × (9 - 1)/2 = 36. In a general case, where there are M sensors i.e. M sensor values in total and each first subgroup contains N sensor values, the number of the first subgroups is M × (M - 1) ×...× (M - N + 1)/(2 × 3 × ... N).

In the exemplifying case illustrated in figure 2, the processing system 102 is configured to record other sensor values S0₁, ..., S0₉ produced by the sensors when air or other gas e.g. ambient air, which is other than the air or other gas carrying the one or more of substances to be detected, is directed to the group of sensors. The processing system 102 is configured to normalize the sensor values S₁, ..., S₉ so that each sensor value after the normalization is the sensor value prior to the normalization divided by the respective recorded other sensor value, i.e. S₁/S0₁, ..., S₉/S0₉. It is also possible, that all sensor values are normalized with a same normalization constant that can be e.g. an average of the sensor values S0₁, ..., S0₉ or some other suitable value. Furthermore, it is also possible that no normalization is carried out. For example, in conjunction with a gas sample analysis, the normalization reduces an adverse effect caused by variation in substances contained by ambient air. In this exemplifying case, the sensor values S0₁, ..., S0₉ can be produced by the sensors when ambient air is directed to the sensors.

The processing system 102 is configured to compute a group of first indicator values Q_{1,2}, ..., Q_{8,9} so that each first indicator value is related to a corresponding one of the first subgroups and is a first pre-determined function of the sensor values belonging to the first subgroup under consideration. In this exemplifying case, the first pre-determined function is a subtraction of the two normalized sensor values belonging to the respective first subgroup: Q_{1,2} = S₁/S0₁ - S₂/S0₂, ... , Q_{3,4} = S₃/S0₃ - S₄/S0₄, ... , Q_{8,9} = S₈/S0₈ - S₉/S0₉. Each first indicator value is associated with index data indicative of the sensors related to the corresponding one of the first subgroups. In the exemplifying case illustrated in figure 2, the index data associated with for example the first indicator value Q₁₂ is the duplet {1, 2} and the index data associated with for example the first indicator value Q_{8,9} is the duplet {8, 9}. Each first indicator value represents a point whose location is determined by the associated index data in a space whose dimension is the number of the sensor values in each first subgroup. In the exemplifying case illustrated in figure 2, each first subgroup contains two sensor values, and thus the space is two-dimensional. In figure 2, the two-dimensional space is denoted with a reference 230. An exemplifying mapping between the index data and locations on the two-dimensional space 230 is shown in figure 2. For example, the location of the point corresponding to the first indicator value Q_{1,2} is the left upper corner of the 6 × 6 grid of the two-dimensional space 230.

The processing system 102 is configured to select a second subgroup from the group of the first indicator values Q_{1,2}, ..., Q_{8,9} in accordance with a first predetermined selection criterion. In this exemplifying case, the first predetermined selection criterion is such that the second subgroup contains a predetermined number of greatest ones of the first indicator values. In the exemplifying case illustrated in figure 2, three greatest first indicator values are selected. In figure 2 shows an exemplifying situation in which Q_{3,4}, Q_{3,9}, and Q_{5,8} are the three greatest first indicator values. The selected second subgroup constitutes a measurement pattern that is depicted with circles in a two-dimensional space 232.

The processing system 102 is configured to compute a distance function between the above-mentioned measurement pattern and a reference pattern corresponding to one or more of the substances. A value of the distance function is indicative of presence or absence of the one or more of the substances, e.g. VOCs. In the exemplifying case illustrated in figure 2, there are two reference patterns in the two-dimensional space 232. The first one of the reference patterns is depicted with diamonds and the second one of the reference patterns is depicted with triangles. In an exemplifying case in which the system is used for detecting a viral infection, the first reference pattern, the diamonds, can represent for example non-infected cases and the second reference pattern, the triangles, can represent for example infected cases. The distance between two squares of the 6 × 6 grid of the two-dimensional space 232 can be defined for example as the number of vertical and/or horizontal and/or diagonal hops needed to move from one of the above-mentioned two squares to the other one.

In a system according to an exemplifying and non-limiting embodiment, the processing system 102 is configured to form pairs each of which comprises one of the points of the measurement pattern and one of points of a reference pattern under consideration, compute pair-specific distances between the two points of each pair based on the locations of the points, and variate the pairing of the points of the measurement pattern and the reference pattern so that a sum of the pair-specific distances is minimized. The minimized sum is the value of the distance function between the measurement pattern and the reference pattern. In the exemplifying situation shown in figure 2, the pairing that provides the smallest distance function between the measurement pattern marked with the circles and the first reference pattern marked with the diamonds is denoted with lines between the circles and the diamonds. With this pairing, the distance function is 1+1+1 = 3. The pairing that provides the smallest distance function between the measurement pattern marked the circles and the second reference pattern marked with the triangles is denoted with lines between the circles and the triangles. With this pairing, the distance function is 1+1+3 = 5 > 3. Thus, in the light of this exemplifying way to define the distance function, the measurement pattern marked with the circles is closer to first reference pattern marked with the diamonds than to the second reference pattern marked with the triangles. Thus, in this exemplifying situation, the result of the detection is: not infected.

In a system according to an exemplifying and non-limiting embodiment, the processing system 102 is configured to compute changes of the sensor values S₁, ..., S₉ with respect to time to produce a group of time-change values. The time-change values may express for example changes of the sensor values during a given time-interval Δt e.g. ΔS₅ or rates of changes during the time-interval e.g. ΔS₅/Δt. The time interval Δt can be for example from 1 second to 10 seconds, e.g. 5 seconds. The processing system 102 is configured to select third subgroups from the group of the time-change values and to compute a group of second indicator values e.g. C_{5,9} based on the third subgroups so that each of the second indicator values is a second pre-determined function of the time-change values belonging to the third subgroup under consideration. In the exemplifying case illustrated in figure 2, the second pre-determined function is a subtraction of the two normalized time-change values belonging to the respective third subgroup e.g. C_{5,9} = ΔS₅/Δt/S0₅ - ΔS₉/Δt/S0₉. Each second indicator value is associated with index data indicative of the sensors related to the corresponding one of the third subgroups. In the exemplifying case illustrated in figure 2, the index data associated with for example the second indicator value C_{2,8} is the duplet {2, 8}. Each second indicator value represents a point whose location is determined by the associated index data in a space whose dimension is the number of the sensor values in each third subgroup. In the exemplifying case illustrated in figure 2, each third subgroup contains two time-change values, and thus the space is two-dimensional. In figure 2, the two-dimensional space is denoted with a reference 231. An exemplifying mapping between the index data and locations on the two-dimensional space 231 is shown in figure 2.

The processing system 102 is configured to select a fourth subgroup from the group of the second indicator values in accordance with a second predetermined selection criterion. In this exemplifying case, the second predetermined selection criterion is such that the fourth subgroup contains a predetermined number of greatest ones of the second indicator values. In the exemplifying case illustrated in figure 2, three greatest second indicator values are selected. Figure 2 shows an exemplifying situation in which C_{2,8}, C_{2,5}, and C_{5,8} are the three greatest second indicator values. The selected fourth subgroup constitutes a measurement pattern that is depicted with circles in a two-dimensional space 233.

The processing system 102 is configured to compute a distance function between the above-mentioned measurement pattern and a reference pattern corresponding to one or more of the substances. A value of the distance function is indicative of presence or absence of the one or more of the substances, e.g. VOCs. In the exemplifying case illustrated in figure 2, there are two reference patterns in the two-dimensional space 233. The first one of the reference patterns is depicted with diamonds and the second one of the reference patterns is depicted with triangles. In an exemplifying case in which the system is used for detecting a viral infection, the first reference pattern, the diamonds, can represent for example non-infected cases and the second reference pattern, the triangles, can represent for example infected cases. The distance between two squares of the 6 × 6 grid in the two-dimensional space 233 can be defined for example as the number of vertical and/or horizontal and/or diagonal hops needed to move from one of the above-mentioned two squares to the other one.

In a system according to an exemplifying and non-limiting embodiment, the processing system 102 is configured compute the distance functions between the measurement pattern and each of the reference patterns on the two-dimensional space 233 in the same way as described above in conjunction with the two-dimensional space 232. In the exemplifying situation shown in figure 2, the pairing that provides the smallest distance function between the measurement pattern marked with the circles in the two-dimensional space 233 and the first reference pattern marked with the diamonds in the two-dimensional space 233 is denoted with lines between the circles and the diamonds. With this pairing, the distance function is 1+1+1 = 3. The pairing that provides the smallest distance function between the measurement pattern marked the circles in the two-dimensional space 233 and the second reference pattern marked with the triangles in the two-dimensional space 233 is denoted with lines between the circles and the triangles. With this pairing, the distance function is 1+1+4 = 6. As mentioned above, the corresponding distance functions in the two-dimensional space 232 are 3 and 5, Thus, the total distance function for the non-infected result candidate, the diamonds, is 3 + 3 = 6, and the total distance function for the infected result candidate, the triangles, is 5 + 6 = 11 > 6. Thus, in the light of this exemplifying way to define the distance function, the result of the detection is: not infected.

The above-described detection technique can be used for detecting a viral load related to an infection such as a viral infection. The viral load, also known as a viral burden, is indicative of quantity of viruses in given volume of fluid, such as sputum fluid. Reference patterns corresponding to different viral loads of a viral infection can be produced based on samples of gas collected from infected living organisms having different known viral loads. The viral loads of these living organisms can be known based on e.g. cycle threshold "Ct" values of a polymerase chain reaction "PCR" tests carried out for these living organisms. In the PCR test, the Ct value is defined as the number of cycles of amplification using the real-time reverse-transcriptase "rRT"-PCR required for the fluorescence of a PCR product to be detected crossing a given threshold which is above a background signal. It is however also possible that the viral loads of the living organisms whose gas samples are used for forming the viral load-specific reference patterns are measured with some other suitable method.

A measurement pattern that has been obtained based on samples of gas collected from a living organism under test is compared to the viral load-specific reference patterns, and the detection result, i.e. the viral load estimate, is determined by which one of the viral load-specific reference patterns is closest to the measurement pattern. In an exemplifying embodiment, the measurement pattern is first compared to two reference patterns one of which corresponds to infected cases and the other one of which corresponds to non-infected cases. Then, if the result of this first detection is infected, the measurement pattern is compared to the viral load-specific reference patterns to obtain an estimate for the viral load. It is also possible to compare the measurement pattern directly to a reference pattern corresponding to non-infected cases and to the viral load-specific reference patterns corresponding to infected cases. In this exemplifying case, the yes/no infection determination and, if there is an infection, the viral load estimation are carried out simultaneously.

It is to be noted that the above-described approach to produce a detection result, e.g. viral non-infected vs. infected, is only a non-limiting example that is presented for illustrative purposes only. For example, the above-mentioned first and/or second predetermined functions can have various forms as well as the above-mentioned first and predetermined selection criteria can have various forms in systems according to different embodiments of the invention. Furthermore, different ways to define the above-mentioned distance function can be used in systems according to different embodiments of the invention.

In a system according to an exemplifying and non-limiting embodiment, the group 101 of sensors comprises one or more sensors that is/are non-responsive to the one or more substances to be detected but responsive to background conditions such as substances other than the substances to be detected and circumstances such as humidity and temperature. The processing system 102 is configured to subtract a compensation value dependent on one or more sensor values produced by the one or more non-responsive sensors from each sensor value produced by the other sensors that are responsive to the one or more substances to be detected. The compensation value can be for example an average of the one or more sensor values produced by the one or more non-responsive sensors. The compensation value can be used for compensating for changes in the background conditions between different measurements. The number of the above-mentioned non-responsive sensors can be for example from 30% to 40% of the total number of the sensors in the group 101 of sensors.

In a system according to an exemplifying and non-limiting embodiment, the group 101 of sensors comprises one or more sensors each being responsive to molecular chirality of one or more of the substances to be detected. The sensitivity to the molecular chirality can be useful when properties of a substance under consideration is dependent on the molecular chirality. A sensor that is responsive to a given molecular chirality can be for example a metal oxide "MOX" sensor having a suitable crystal structure chirality in its sensor layer whose electrical resistance is dependent on presence of a certain substance having the given molecular chirality.

In a system according to an exemplifying and non-limiting embodiment, the processing system 102 is configured to compute the distance function between the measurement pattern and each of two or more reference patterns each of which corresponds to a substance combination comprising one or more substances. For example, different ones of the substance combinations may correspond to different states of a living organism. Each value of the distance function is indicative of presence or absence of the corresponding substance combination, e.g. presence or absence of the corresponding state of a living organism.

In a system according to an exemplifying and non-limiting embodiment, the reference pattern expresses reference ratios between concentrations of the substances and the distance function between the measurement pattern and the reference pattern is indicative of differences between concentration ratios of the substances and the reference ratios. The reference ratios can be understood to express a reference shape of a histogram of the concentrations and the concentration ratios can be understood to express a detected shape of the histogram of the concentrations. In many cases, a mere presence of one or more substances is not necessarily a sufficient indicator for a given state of a living organism, but the state of a living organism can be detected based on ratios between concentrations of the substances i.e. the shape of a concentration histogram of the substances.

In a system according to an exemplifying and non-limiting embodiment, the processing system 102 is configured to change temperatures of the sensors to change the acceptances of the sensors for the substances. For example, a metal oxide "MOX" sensor has a heater plate under a sensor layer and an operating temperature range can be e.g. from 150°C to 400°C. The acceptance of the MOX sensor for different substances can be changed by changing the temperature. The implementation of the controller 109 can be based on one or more processor circuits, each of which can be a programmable processor circuit provided with appropriate software, a dedicated hardware processor such as for example an application specific integrated circuit "ASIC", or a configurable hardware processor such as for example a field programmable gate array "FPGA". Furthermore, the controller 109 may comprise one or more memory devices such as e.g. random-access memory "RAM" circuits. Correspondingly, the implementation of the processing system 102 can be based on one or more processor circuits, each of which can be a programmable processor circuit provided with appropriate software, a dedicated hardware processor such as for example an application specific integrated circuit "ASIC", or a configurable hardware processor such as for example a field programmable gate array "FPGA". Furthermore, the processing system 102 may comprise one or more memory devices such as e.g. random-access memory "RAM" circuits.

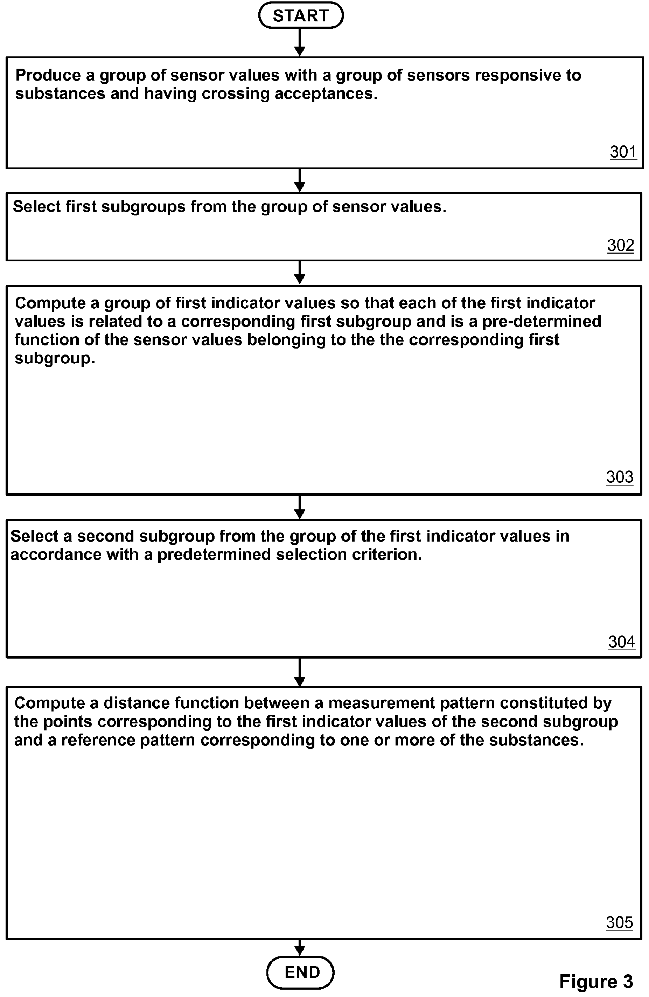

Figure 3 shows a flowchart of the method of claim 14 for detecting one or more substances, e.g. VOCs, carried by air or other gas. The method comprises the following actions:
- action 301: producing a group of sensor values with a group of sensors responsive to many substances, the sensors having crossing acceptances for the substances so that different ones of the sensors have maximum acceptances for different ones of the substances,
- action 302: selecting first subgroups from the group of sensor values, the first subgroups being different from each other,
- action 303: computing a group of first indicator values so that each of the first indicator values is related to a corresponding one of the first subgroups and is a first pre-determined function of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- action 304: selecting a second subgroup from the group of the first indicator values in accordance with a first predetermined selection criterion, and
- action 305: computing a distance function between a measurement pattern constituted by at least the points corresponding to the first indicator values belonging to the second subgroup and a reference pattern corresponding to one or more of the substances, a value of the distance function being indicative of presence or absence of the one or more of the substances.

In an example, each of the first subgroups can contain two of the sensor values and the first pre-determined function comprises a subtraction of the two of the sensor values.

The first predetermined selection criterion can be such that the second subgroup contains a predetermined number of greatest ones of the first indicator values.

The method can further comprise:
- computing changes of the sensor values with respect to time to produce a group of time-change values,
- selecting third subgroups from the group of time-change values,
- computing a group of second indicator values based on the third subgroups so that each of the second indicator values is a second pre-determined function of the time-change values belonging to the third subgroup under consideration,
- selecting a fourth subgroup from the group of the second indicator values in accordance with a second predetermined selection criterion, and
- computing the distance function between the reference pattern and the measurement pattern constituted by the points corresponding to the first indicator values belonging to the second subgroup and points corresponding to the second indicator values belonging to the fourth subgroup.

Each of the third subgroups can contain two of the time-change values and the second pre-determined function comprises a subtraction of the two of the time-change values.

The second predetermined selection criterion can be such that the fourth subgroup contains a predetermined number of greatest ones of the second indicator values.

The method can further comprise:
- forming pairs each comprising one of the points of the measurement pattern and one of points of the reference pattern,
- computing pair-specific distances between the two points of each pair based on the locations of the points, and
- variating the pairing of the points of the measurement pattern and the reference pattern so that a sum of the pair-specific distances is minimized, the minimized sum being the value of the distance function.

The reference pattern corresponds to a mixture of two or more of the substances.

The method can further comprise:
- recording other sensor values produced by the sensors when air or other gas, other than the air or the other gas carrying the one or more substances to be detected, is directed to the group of sensors, and
- normalizing the sensor values so that each sensor value after the normalization is the sensor value prior to the normalization divided by the respective recorded other sensor value.

The group of sensors can comprise one or more sensors non-responsive to the one or more substances to be detected, and the method comprises subtracting a compensation value dependent on one or more sensor values produced by the one or more non-responsive sensors from each sensor value produced by the other sensors responsive to the one or more substances to be detected.

The group of sensors can comprise one or more sensors each being responsive to molecular chirality of one or more of the substances to be detected.

Is possible to compute the distance function between the measurement pattern and each of two or more reference patterns each corresponding to a substance combination comprising one or more of the substances, where each value of the distance function is indicative of presence or absence of the corresponding substance combination.

The reference pattern expresses reference ratios between concentrations of the substances and the distance function between the measurement pattern and the reference pattern is indicative of differences between concentration ratios of the substances and the reference ratios.

Temperatures of the sensors can be changed to change the acceptances of the sensors for the substances.

A computer program can be employed which comprises computer executable instructions for controlling a programmable processing system to carry out actions related to a method according to any of the above-described exemplifying and non-limiting embodiments.

A computer program according to claim 15 comprises software modules for detecting one or more substances, e.g. VOCs, carried by air or other gas. The software modules comprise computer executable instructions for controlling a programmable processing system to:
- receive a group of sensor values from a group of sensors responsive to many substances, the sensors having crossing acceptances for the substances so that different ones of the sensors have maximum acceptances for different ones of the substances,
- select first subgroups from the group of sensor values, the first subgroups being different from each other,
- compute a group of first indicator values so that each of the first indicator values is related to a corresponding one of the first subgroups and is a first pre-determined function of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- select a second subgroup from the group of the first indicator values in accordance with a first predetermined selection criterion, and
- compute a distance function between a measurement pattern constituted by at least the points corresponding to the first indicator values belonging to the second subgroup and a reference pattern corresponding to one or more of the substances, a value of the distance function being indicative of presence or absence of the one or more of the substances.

The software modules can be for example subroutines or functions implemented with programming tools suitable for the programmable processing system.

A computer program product can be provided which comprises a non-transitory computer readable medium, e.g. a compact disc "CD", encoded with a computer program according to an exemplifying embodiment.

A signal can be encoded to carry information defining a computer program according to an exemplifying embodiment.

Such a computer program may constitute a part of a software of a device such as a smart phone or a wearable device.

The specific examples provided in the description given above should not be construed as limiting the applicability and/or interpretation of the appended claims. It is to be noted that lists and groups of examples given in this document are non-exhaustive lists and groups unless otherwise explicitly stated.

## Claims

1. A system for detecting one or more of substances carried by air or other gas, the system comprising:
- a group (101) of sensors (103, 104) responsive to the substances to produce a group of sensor values (S₁, ..., S₉), the sensors having crossing acceptances for the substances so that different ones of the sensors have maximum acceptances for different ones of the substances,
**characterized in that** the system comprises a processing system (102) configured to:
- select first subgroups from the group of sensor values, the first subgroups being different from each other,
- compute a group of first indicator values (Q_{1,2}, ..., Q_{8,9}) so that each of the first indicator values is related to a corresponding one of the first subgroups and is a first pre-determined function (S₁/S0₁ - S₂/S0₂) of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- select a second subgroup from the group of the first indicator values in accordance with a first predetermined selection criterion,
- compute a distance function between a measurement pattern constituted by at least the points corresponding to the first indicator values belonging to the second subgroup and a reference pattern corresponding to one or more of the substances, a value of the distance function being indicative of presence or absence of the one or more of the substances.

2. A system according to claim 1, wherein each of the first subgroups contains two of the sensor values and the first pre-determined function comprises a subtraction (S₁/S0₁ - S₂/S0₂) of the two of the sensor values.

3. A system according to claim 1 or 2, wherein the first predetermined selection criterion is such that the second subgroup contains a predetermined number of greatest ones of the first indicator values.

4. A system according to any one of claims 1-3, wherein the processing system (102) is configured to:
- compute changes of the sensor values with respect to time to produce a group of time-change values (..., ΔS₅/Δt, ..., ΔS₉/Δt),
- select third subgroups from the group of time-change values,
- compute a group of second indicator values (..., C_{2,5}, ... C_{5,9}, ... ) based on the third subgroups so that each of the second indicator values is a second pre-determined function (Δ_{S5}/Δt/S0₁ - ΔS₉/Δt/S0₂) of the time-change values belonging to the third subgroup under consideration,
- select a fourth subgroup from the group of the second indicator values in accordance with a second predetermined selection criterion, and
- compute the distance function between the reference pattern and the measurement pattern constituted by the points corresponding to the first indicator values belonging to the second subgroup and points corresponding to the second indicator values belonging to the fourth subgroup.

5. A system according to claim 4, wherein each of the third subgroups contains two of the time-change values and the second pre-determined function (ΔS₅/Δt/S0₁ - ΔS₉/Δt/S0₂) comprises a subtraction of the two of the time-change values.

6. A system according to any one of claims 1-5, wherein the processing system (102) is configured to:
- form pairs each comprising one of the points of the measurement pattern and one of points of the reference pattern,
- compute pair-specific distances between the two points of each pair based on the locations of the points, and
- variate the pairing of the points of the measurement pattern and the reference pattern so that a sum of the pair-specific distances is minimized, the minimized sum being the value of the distance function.

7. A system according to any one of claims 1-6, wherein the reference pattern corresponds to a mixture of two or more of the substances.

8. A system according to any one of claims 1-7, wherein the processing system (102) is configured to:
- record other sensor values (S0₁, S0₂) produced by the sensors when air or other gas, other than the air or the other gas carrying the one or more of substances to be detected, is directed to the group of sensors, and
- normalize the sensor values (S₁, S₂) so that each sensor value after the normalization is the sensor value prior to the normalization divided by the respective recorded other sensor value.

9. A system according to any one of claims 1-8, wherein the group (101) of sensors (103, 104) comprises one or more sensors non-responsive to the one or more substances to be detected, and the processing system (102) is configured to subtract a compensation value dependent on one or more sensor values produced by the one or more non-responsive sensors from each sensor value produced by other ones of the sensors responsive to the one or more substances to be detected.

10. A system according to any one of claims 1-9, wherein the group (101) of sensors (103, 104) comprises one or more sensors each being responsive to molecular chirality of one or more of the substances to be detected.

11. A system according to any one of claims 1-10, wherein the processing system (102) is configured to compute the distance function between the measurement pattern and each of two or more reference patterns each corresponding to a substance combination comprising one or more of the substances, each value of the distance function being indicative of presence or absence of the corresponding substance combination.

12. A system according to any one of claims 1-11, wherein the reference pattern expresses reference ratios between concentrations of the substances and the distance function between the measurement pattern and the reference pattern is indicative of differences between concentration ratios of the substances and the reference ratios.

13. A system according to any one of claims 1-12, wherein the processing system (102) is configured to change temperatures of the sensors to change the acceptances of the sensors for the substances.

14. A method for detecting one or more of substances carried by air or other gas, the method comprising:
- producing (301) a group of sensor values (S₁, ..., S₉) with a group of sensors responsive to the substances, the sensors having crossing acceptances for the substances so that different ones of the sensors have maximum acceptances for different ones of the substances,
**characterized in that** the method comprises:
- selecting (302) first subgroups from the group of sensor values, the first subgroups being different from each other,
- computing (303) a group of first indicator values (Q_{1,2}, ..., Q_{8,9}) so that each of the first indicator values is related to a corresponding one of the first subgroups and is a first pre-determined function (S₁/S0₁ - S₂/S0₂) of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- selecting (304) a second subgroup from the group of the first indicator values in accordance with a first predetermined selection criterion, and
- computing (305) a distance function between a measurement pattern constituted by at least the points corresponding to the first indicator values belonging to the second subgroup and a reference pattern corresponding to one or more of the substances, a value of the distance function being indicative of presence or absence of the one or more of the substances.

15. A computer program for detecting one or more of substances carried by air, the computer program comprising computer executable instructions for controlling a programmable processing system to:
- receive a group of sensor values (S₁, ..., S₉) from a group of sensors responsive to the substances, the sensors having crossing acceptances for the substances so that different ones of the sensors have maximum acceptances for different ones of the substances,
**characterized in that** the computer program further comprises computer executable instructions for controlling the programmable processing system to:
- select first subgroups from the group of sensor values, the first subgroups being different from each other,
- compute a group of first indicator values (Q_{1,2}, ..., Q_{8,9}) so that each of the first indicator values is related to a corresponding one of the first subgroups and is a first pre-determined function of the sensor values belonging to the first subgroup under consideration, each of the first indicator values i) being associated with index data indicative of the sensors belonging to the corresponding one of the first subgroups and ii) representing a point whose location is determined by the associated index data in a space whose dimension is a number of the sensor values in each of the first subgroups,
- select a second subgroup from the group of the first indicator values in accordance with a first predetermined selection criterion, and
- compute a distance function between a measurement pattern constituted by at least the points corresponding to the first indicator values belonging to the second subgroup and a reference pattern corresponding to one or more of the substances, a value of the distance function being indicative of presence or absence of the one or more of the substances.

## Patentansprüche

1. System zur Erkennung von einer oder mehreren Substanzen, die von Luft oder einem anderen Gas mitgeführt werden, wobei das System Folgendes umfasst:
- eine Gruppe (101) von Sensoren (103, 104), die auf die Substanzen ansprechen, zum Erzeugen einer Gruppe von Sensorwerten (S₁, ..., S₉), wobei die Sensoren sich überschneidende Empfindlichkeiten für die Substanzen aufweisen, sodass unterschiedliche der Sensoren maximale Empfindlichkeiten für unterschiedliche der Substanzen aufweisen,
**dadurch gekennzeichnet, dass** das System ein Verarbeitungssystem (102) umfasst, das zu Folgendem konfiguriert ist:
- Auswählen erster Untergruppen aus der Gruppe von Sensorwerten, wobei sich die ersten Untergruppen voneinander unterscheiden,
- Berechnen einer Gruppe von ersten Indikatorwerten (Q_{1,2}, ..., Q_{8,9}), sodass jeder der ersten Indikatorwerte mit einer entsprechenden der ersten Untergruppen in Beziehung steht und eine erste vorbestimmte Funktion (S₁/S0₁ - S₂/S0₂) der Sensorwerte ist, die zu der betrachteten ersten Untergruppe gehören, wobei jeder der ersten Indikatorwerte i) mit Indexdaten assoziiert ist, die Aufschluss über die Sensoren geben, die zu der entsprechenden der ersten Untergruppen gehören, und ii) einen Punkt darstellt, dessen Position durch die assoziierten Indexdaten in einem Raum bestimmt wird, dessen Dimension einer Anzahl der Sensorwerte in jeder der ersten Untergruppen entspricht,
- Auswählen einer zweiten Untergruppe aus der Gruppe der ersten Indikatorwerte in Übereinstimmung mit einem ersten vorbestimmten Auswahlkriterium,
- Berechnen einer Abstandsfunktion zwischen einem Messmuster, das mindestens aus den Punkten besteht, die den ersten Indikatorwerten entsprechen, die zu der zweiten Untergruppe gehören, und einem Referenzmuster, das einer oder mehreren der Substanzen entspricht, wobei ein Wert der Abstandsfunktion Aufschluss über das Vorhandensein oder die Abwesenheit der einen oder mehreren der Substanzen gibt.

2. System nach Anspruch 1, wobei jede der ersten Untergruppen zwei der Sensorwerte enthält und die erste vorbestimmte Funktion eine Subtraktion (S₁/S0₁ - S₂/S0₂) der beiden Sensorwerte umfasst.

3. System nach Anspruch 1 oder 2, wobei das erste vorbestimmte Auswahlkriterium derart ist, dass die zweite Untergruppe eine vorbestimmte Anzahl der größten ersten Indikatorwerte enthält.

4. System nach einem der Ansprüche 1-3, wobei das Verarbeitungssystem (102) zu Folgendem konfiguriert ist:
- Berechnen von Veränderungen der Sensorwerte im Laufe der Zeit zum Erzeugen einer Gruppe von Zeitänderungswerten (..., ΔS₅/Δt, ..., ΔS₉/Δt),
- Auswählen dritter Untergruppen aus der Gruppe von Zeitänderungswerten,
- Berechnen einer Gruppe von zweiten Indikatorwerten (..., C_{2,5}, ... C_{5,9}, ...) basierend auf den dritten Untergruppen, sodass jeder der zweiten Indikatorwerte eine zweite vorbestimmte Funktion (ΔS₅/Δt/S0₁ - ΔS₉/Δt/S0₂) der Zeitänderungswerte ist, die zu der betrachteten dritten Untergruppe gehören,
- Auswählen einer vierten Untergruppe aus der Gruppe der zweiten Indikatorwerte in Übereinstimmung mit einem zweiten vorbestimmten Auswahlkriterium, und
- Berechnen der Abstandsfunktion zwischen dem Referenzmuster und dem Messmuster, das aus den Punkten besteht, die den ersten Indikatorwerten entsprechen, die zur zweiten Untergruppe gehören, sowie Punkten, die den zweiten Indikatorwerten entsprechen, die zur vierten Untergruppe gehören.

5. System nach Anspruch 4, wobei jede der dritten Untergruppen zwei der Zeitänderungswerte enthält und die zweite vorbestimmte Funktion (ΔS₅/Δt/S0₁ - ΔS₉/Δt/S0₂) eine Subtraktion der beiden Zeitänderungswerte umfasst.

6. System nach einem der Ansprüche 1-5, wobei das Verarbeitungssystem (102) zu Folgendem konfiguriert ist:
- Bilden von Paaren, welche jeweils einen der Punkte des Messmusters und einen der Punkte des Referenzmusters umfassen,
- Berechnen von paarspezifischen Abständen zwischen den zwei Punkten jedes Paares basierend auf den Positionen der Punkte, und
- Variieren der Paarung der Punkte des Messmusters und des Referenzmusters, sodass eine Summe der paarspezifischen Abstände minimiert wird, wobei die minimierte Summe den Wert der Abstandsfunktion darstellt.

7. System nach einem der Ansprüche 1-6, wobei das Referenzmuster einer Mischung aus zwei oder mehr der Substanzen entspricht.

8. System nach einem der Ansprüche 1-7, wobei das Verarbeitungssystem (102) zu Folgendem konfiguriert ist:
- Erfassen anderer Sensorwerte (S0₁, S0₂), die durch die Sensoren erzeugt werden, wenn andere Luft oder ein anderes Gas als die Luft oder das andere Gas, welche die eine oder die mehreren der zu erkennenden Substanzen mitführen, zu der Gruppe von Sensoren geleitet werden, und
- Normalisieren der Sensorwerte (S₁, S₂), sodass jeder Sensorwert nach der Normalisierung dem Sensorwert vor der Normalisierung geteilt durch den entsprechenden erfassten anderen Sensorwert entspricht.

9. System nach einem der Ansprüche 1-8, wobei die Gruppe (101) von Sensoren (103, 104) einen oder mehrere Sensoren umfasst, die nicht auf die eine oder die mehreren zu erkennenden Substanzen ansprechen, und das Verarbeitungssystem (102) konfiguriert ist zum Subtrahieren eines Ausgleichswertes in Abhängigkeit von einem oder mehreren Sensorwerten, die durch den einen oder die mehreren nicht-ansprechenden Sensoren erzeugt werden, von jedem Sensorwert, der durch andere der Sensoren, die auf die eine oder die mehreren zu erkennenden Substanzen ansprechen, erzeugt wird.

10. System nach einem der Ansprüche 1-9, wobei die Gruppe (101) von Sensoren (103, 104) einen oder mehrere Sensoren umfasst, welche jeweils auf eine molekulare Chiralität der einen oder der mehreren zu erkennenden Substanzen ansprechen.

11. System nach einem der Ansprüche 1-10, wobei das Verarbeitungssystem (102) konfiguriert ist zum Berechnen der Abstandsfunktion zwischen dem Messmuster und jedem von zwei oder mehr Referenzmustern, die jeweils einer Substanzkombination entsprechen, welche eine oder mehrere der Substanzen umfasst, wobei jeder Wert der Abstandsfunktion Aufschluss über das Vorhandensein oder die Abwesenheit der entsprechenden Substanzkombination gibt.

12. System nach einem der Ansprüche 1-11, wobei das Referenzmuster Referenzverhältnisse zwischen Konzentrationen der Substanzen ausdrückt und die Abstandsfunktion zwischen dem Messmuster und dem Referenzmuster Aufschluss über Unterschiede zwischen Konzentrationsverhältnissen der Substanzen und den Referenzverhältnissen gibt.

13. System nach einem der Ansprüche 1-12, wobei das Verarbeitungssystem (102) zum Verändern von Temperaturen der Sensoren konfiguriert ist, um die Empfindlichkeiten der Sensoren für die Substanzen anzupassen.

14. Verfahren zur Erkennung von einer oder mehreren Substanzen, die von Luft oder einem anderen Gas mitgeführt werden, wobei das Verfahren Folgendes umfasst:
- Erzeugen (301) einer Gruppe von Sensorwerten (S₁, ..., S₉) mit einer Gruppe von Sensoren, die auf die Substanzen ansprechen, wobei die Sensoren sich überschneidende Empfindlichkeiten für die Substanzen aufweisen, sodass unterschiedliche der Sensoren maximale Empfindlichkeiten für unterschiedliche der Substanzen aufweisen,
**dadurch gekennzeichnet, dass** das Verfahren Folgendes umfasst:
- Auswählen (302) erster Untergruppen aus der Gruppe von Sensorwerten, wobei sich die ersten Untergruppen voneinander unterscheiden,
- Berechnen (303) einer Gruppe von ersten Indikatorwerten (Q_{1,2}, ..., Q_{8,9}), sodass jeder der ersten Indikatorwerte mit einer entsprechenden der ersten Untergruppen in Beziehung steht und eine erste vorbestimmte Funktion (S₁/S0₁ - S₂/S0₂) der Sensorwerte ist, die zu der betrachteten ersten Untergruppe gehören, wobei jeder der ersten Indikatorwerte i) mit Indexdaten assoziiert ist, die Aufschluss über die Sensoren geben, die zu der entsprechenden der ersten Untergruppen gehören, und ii) einen Punkt darstellt, dessen Position durch die assoziierten Indexdaten in einem Raum bestimmt wird, dessen Dimension einer Anzahl der Sensorwerte in jeder der ersten Untergruppen entspricht,
- Auswählen (304) einer zweiten Untergruppe aus der Gruppe der ersten Indikatorwerte in Übereinstimmung mit einem ersten vorbestimmten Auswahlkriterium, und
- Berechnen (305) einer Abstandsfunktion zwischen einem Messmuster, das mindestens aus den Punkten besteht, die den ersten Indikatorwerten entsprechen, die zu der zweiten Untergruppe gehören, und einem Referenzmuster, das einer oder mehreren der Substanzen entspricht, wobei ein Wert der Abstandsfunktion Aufschluss über das Vorhandensein oder die Abwesenheit der einen oder mehreren der Substanzen gibt.

15. Computerprogramm zur Erkennung von einer oder mehreren Substanzen, die von Luft mitgeführt werden, wobei das Computerprogramm computerausführbare Anweisungen zur Steuerung eines programmierbaren Verarbeitungssystems für Folgendes umfasst:
- Empfangen einer Gruppe von Sensorwerten (S₁, ..., S₉) von einer Gruppe von Sensoren, die auf die Substanzen ansprechen, wobei die Sensoren sich überschneidende Empfindlichkeiten für die Substanzen aufweisen, sodass unterschiedliche der Sensoren maximale Empfindlichkeiten für unterschiedliche der Substanzen aufweisen,
**dadurch gekennzeichnet, dass** das Computerprogramm ferner computerausführbare Anweisungen zur Steuerung des programmierbaren Verarbeitungssystems für Folgendes umfasst:
- Auswählen erster Untergruppen aus der Gruppe von Sensorwerten, wobei sich die ersten Untergruppen voneinander unterscheiden,
- Berechnen einer Gruppe von ersten Indikatorwerten (Q_{1,2}, ..., Q_{8,9}), sodass jeder der ersten Indikatorwerte mit einer entsprechenden der ersten Untergruppen in Beziehung steht und eine erste vorbestimmte Funktion der Sensorwerte ist, die zu der betrachteten ersten Untergruppe gehören, wobei jeder der ersten Indikatorwerte i) mit Indexdaten assoziiert ist, die Aufschluss über die Sensoren geben, die zu der entsprechenden der ersten Untergruppen gehören, und ii) einen Punkt darstellt, dessen Position durch die assoziierten Indexdaten in einem Raum bestimmt wird, dessen Dimension einer Anzahl der Sensorwerte in jeder der ersten Untergruppen entspricht,
- Auswählen einer zweiten Untergruppe aus der Gruppe der ersten Indikatorwerte in Übereinstimmung mit einem ersten vorbestimmten Auswahlkriterium, und
- Berechnen einer Abstandsfunktion zwischen einem Messmuster, das mindestens aus den Punkten besteht, die den ersten Indikatorwerten entsprechen, die zu der zweiten Untergruppe gehören, und einem Referenzmuster, das einer oder mehreren der Substanzen entspricht, wobei ein Wert der Abstandsfunktion Aufschluss über das Vorhandensein oder die Abwesenheit der einen oder mehreren der Substanzen gibt.

## Revendications

1. Système de détection d'une ou plusieurs substances véhiculées par de l'air ou un autre gaz, le système comprenant :
- un groupe (101) de capteurs (103, 104) sensibles aux substances pour produire un groupe de valeurs de capteur (S₁,..., S₉), les capteurs présentant des acceptances qui se recoupent pour les substances, de sorte que différents capteurs présentent des acceptances maximales pour différentes substances,
**caractérisé en ce que** le système comprend un système de traitement (102) configuré pour :
- sélectionner des premiers sous-groupes parmi le groupe de valeurs de capteur, les premiers sous-groupes étant différents les uns des autres,
- calculer un groupe de premières valeurs indicatrices (Q_{1,2},..., Q_{8,9}) de telle sorte que chacune des premières valeurs indicatrices soit associée à un sous-groupe correspondant parmi les premiers sous-groupes et constitue une première fonction prédéterminée (S₁/S0₁-S₂/S0₂) des valeurs de capteur appartenant au premier sous-groupe considéré, chacune des premières valeurs indicatrices i) étant associée à des données d'indice indiquant les capteurs appartenant au sous-groupe correspondant parmi les premiers sous-groupes et ii) représentant un point dont la position est déterminée par les données d'indice associées dans un espace dont la dimension correspond à un nombre de valeurs de capteur dans chacun des premiers sous-groupes,
- sélectionner un deuxième sous-groupe parmi le groupe des premières valeurs indicatrices conformément à un premier critère de sélection prédéterminé,
- calculer une fonction de distance entre un motif de mesure constitué par au moins les points correspondant aux premières valeurs indicatrices appartenant au deuxième sous-groupe et un motif de référence correspondant à une ou plusieurs des substances, une valeur de la fonction de distance étant indicative de la présence ou de l'absence d'une ou plusieurs des substances.

2. Système selon la revendication 1, dans lequel chacun des premiers sous-groupes contient deux des valeurs de capteur et la première fonction prédéterminée comprend une soustraction (S₁/S0₁-S₂/S0₂) de deux des valeurs de capteur.

3. Système selon la revendication 1 ou 2, dans lequel le premier critère de sélection prédéterminé est tel que le deuxième sous-groupe contient un nombre prédéterminé des plus grandes valeurs parmi les premières valeurs indicatrices.

4. Système selon une quelconque des revendications 1 à 3, dans lequel le système de traitement (102) est configuré pour :
- calculer des variations des valeurs de capteur par rapport au temps pour produire un groupe de valeurs de variation temporelle (..., ΔS₅/Δt,..., ΔS₉/Δt),
- sélectionner des troisièmes sous-groupes parmi le groupe de valeurs de variation temporelle,
- calculer un groupe de deuxièmes valeurs d'indicateur (..., C_{2,5},..., C_{5,9},...) sur la base des troisièmes sous-groupes de telle sorte que chacune des deuxièmes valeurs d'indicateur soit une deuxième fonction prédéterminée (ΔS₅/Δt/S0₁-ΔS₉/Δt/S0₂) des valeurs de variation temporelle appartenant au troisième sous-groupe considéré,
- sélectionner un quatrième sous-groupe parmi le groupe des deuxièmes valeurs d'indicateur conformément à un deuxième critère de sélection prédéterminé et
- calculer la fonction de distance entre le motif de référence et le motif de mesure constitué par les points correspondant aux premières valeurs d'indicateur appartenant au deuxième sous-groupe et les points correspondant aux deuxièmes valeurs d'indicateur appartenant au quatrième sous-groupe.

5. Système selon la revendication 4, dans lequel chacun des troisièmes sous-groupes contient deux des valeurs de variation temporelle et la deuxième fonction prédéterminée (ΔS₅/Δt/S0₁-ΔS₉/Δt/S0₂) comprend une soustraction de deux des valeurs de variation temporelle.

6. Système selon une quelconque des revendications 1 à 5, dans lequel le système de traitement (102) est configuré pour :
- former des paires comprenant chacune un des points du motif de mesure et un des points du motif de référence,
- calculer des distances spécifiques aux paires entre les deux points de chaque paire sur la base des positions des points et
- faire varier l'appariement des points du motif de mesure et du motif de référence de telle sorte qu'une somme des distances spécifiques aux paires soit minimisée, la somme minimisée constituant la valeur de la fonction de distance.

7. Système selon une quelconque des revendications 1 à 6, dans lequel le motif de référence correspond à un mélange de deux ou plusieurs des substances.

8. Système selon une quelconque des revendications 1 à 7, dans lequel le système de traitement (102) est configuré pour :
- enregistrer d'autres valeurs de capteur (S0₁, S0₂) produites par les capteurs lorsque de l'air ou un autre gaz, distinct de l'air ou de l'autre gaz transportant une ou plusieurs substances à détecter, est dirigé vers le groupe de capteurs et
- normaliser les valeurs de capteur (S₁, S₂) de telle sorte que chaque valeur de capteur après la normalisation soit la valeur de capteur avant la normalisation divisée par l'autre valeur de capteur enregistrée respective.

9. Système selon une quelconque des revendications 1 à 8, dans lequel le groupe (101) de capteurs (103, 104) comprend un ou plusieurs capteurs insensibles à une ou plusieurs substances à détecter et le système de traitement (102) est configuré pour soustraire une valeur de compensation dépendant d'une ou plusieurs valeurs de capteur produites par un ou plusieurs capteurs insensibles de chaque valeur de capteur produite par d'autres parmi les capteurs sensibles à une ou plusieurs substances à détecter.

10. Système selon une quelconque des revendications 1 à 9, dans lequel le groupe (101) de capteurs (103, 104) comprend un ou plusieurs capteurs étant chacun sensible à la chiralité moléculaire d'une ou plusieurs des substances à détecter.

11. Système selon une quelconque des revendications 1 à 10, dans lequel le système de traitement (102) est configuré pour calculer la fonction de distance entre le motif de mesure et chacun de deux motifs de référence ou plus, correspondant chacun à une combinaison de substances comprenant une ou plusieurs des substances, chaque valeur de la fonction de distance étant indicative de la présence ou de l'absence de la combinaison de substances correspondante.

12. Système selon une quelconque des revendications 1 à 11, dans lequel le motif de référence exprime des rapports de référence entre les concentrations des substances et la fonction de distance entre le motif de mesure et le motif de référence est indicative de différences entre les rapports de concentration des substances et les rapports de référence.

13. Système selon une quelconque des revendications 1 à 12, dans lequel le système de traitement (102) est configuré pour modifier les températures des capteurs afin de modifier les acceptances des capteurs vis-à-vis des substances.

14. Procédé de détection d'une ou plusieurs substances véhiculées par de l'air ou un autre gaz, le procédé comprenant :
- la production (301) d'un groupe de valeurs de capteur (S₁,..., S₉) au moyen d'un groupe de capteurs sensibles aux substances, les capteurs présentant des acceptances qui se recoupent pour les substances, de sorte que différents capteurs présentent des réponses maximales pour différentes substances,
**caractérisé en ce que** le procédé comprend :
- la sélection (302) de premiers sous-groupes parmi le groupe de valeurs de capteur, les premiers sous-groupes étant différents les uns des autres,
- le calcul (303) un groupe de premières valeurs indicatrices (Q_{1,2},..., Q_{8,9}) de telle sorte que chacune des premières valeurs indicatrices soit associée à un sous-groupe correspondant parmi les premiers sous-groupes et constitue une première fonction prédéterminée (S₁/S0₁-S₂/S0₂) des valeurs de capteur appartenant au premier sous-groupe considéré, chacune des premières valeurs indicatrices i) étant associée à des données d'indice indiquant les capteurs appartenant au sous-groupe correspondant parmi les premiers sous-groupes et ii) représentant un point dont la position est déterminée par les données d'indice associées dans un espace dont la dimension correspond à un nombre de valeurs de capteur dans chacun des premiers sous-groupes,
- la sélection (304) d'un deuxième sous-groupe parmi le groupe des premières valeurs indicatrices conformément à un premier critère de sélection prédéterminé et
- le calcul (305) d'une fonction de distance entre un motif de mesure, constitué au moins par les points correspondant aux premières valeurs indicatrices appartenant au deuxième sous-groupe et un motif de référence correspondant à une ou plusieurs des substances, une valeur de la fonction de distance étant indicative de la présence ou de l'absence d'une ou plusieurs des substances.

15. Programme informatique pour détecter une ou plusieurs substances véhiculées par del'air, le programme informatique comprenant des instructions exécutables par ordinateur pour commander un système de traitement programmable afin de :
- recevoir un groupe de valeurs de capteurs (S₁,..., S₉) provenant d'un groupe de capteurs sensibles aux substances, les capteurs présentant des acceptances qui se recoupent pour les substances, de sorte que différents capteurs présentent des acceptances maximales pour différentes substances parmi les substances,
**caractérisé en ce que** le programme informatique comprend en outre des instructions exécutables par ordinateur pour commander le système de traitement programmable afin de :
- sélectionner des premiers sous-groupes parmi le groupe de valeurs de capteurs, les premiers sous-groupes étant différents les uns des autres,
- calculer un groupe de premières valeurs indicatrices (Q_{1,2},..., Q_{8,9}) de telle sorte que chacune des premières valeurs indicatrices soit associée à un sous-groupe correspondant parmi les premiers sous-groupes et constitue une première fonction prédéterminée des valeurs de capteur appartenant au premier sous-groupe considéré, chacune des premières valeurs indicatrices i) étant associée à des données d'indice indiquant les capteurs appartenant au sous-groupe correspondant parmi les premiers sous-groupes et ii) représentant un point dont la position est déterminée par les données d'indice associées dans un espace dont la dimension correspond à un nombre de valeurs de capteur dans chacun des premiers sous-groupes,
- sélectionner un deuxième sous-groupe parmi le groupe des premières valeurs indicatrices conformément à un premier critère de sélection prédéterminé et
- calculer une fonction de distance entre un motif de mesure, constitué au moins par les points correspondant aux premières valeurs indicatrices appartenant au deuxième sous-groupe et un motif de référence correspondant à une ou plusieurs des substances, une valeur de la fonction de distance étant indicative de la présence ou de l'absence d'une ou plusieurs des substances.
